# EUROPÄISCHE PATENTSCHRIFT

(11) **EP 1 159 405 B1**
(45) Veröffentlichungstag und Bekanntmachung des Hinweises auf die Patenterteilung: **20.06.2007**
(21) Anmeldenummer: 00916891.5
(22) Anmeldetag: 02.03.2000
(51) Int. Cl.: C12N 7/00, A61K 39/245, C12N 15/38, A61P 31/22

(54) **VIRALE PARTIKEL, DIE NACH INFEKTION DURCH HUMANES CYTOMEGALOVIRUS FREIGESETZT WERDEN UND IHRE VERWENDUNG ALS IMPFSTOFF**
VIRAL PARTICLES WHICH ARE RELEASED AFTER THE INFECTION WITH THE HUMAN CYTOMEGALOVIRUS AND THE USE OF SAID PARTICLES AS A VACCINE
PARTICULES VIRALES LIBEREES APRES INFECTION PAR LE VIRUS CYTOMEGALIQUE HUMAIN ET LEUR UTILISATION COMME VACCIN

(30) Priorität: 08.03.1999 DE 19910044
(43) Veröffentlichungstag der Anmeldung: 05.12.2001
(73) Patentinhaber: Plachter, Bodo, 55286 Wörrstadt (DE)
(72) Erfinder: Plachter, Bodo, 55286 Wörrstadt (DE)
(74) Vertreter: Keller, Günter
(86) Internationale Anmeldenummer: PCT/EP2000/001794
(87) Internationale Veröffentlichungsnummer: WO 2000/053729

(56) Entgegenhaltungen:
- WO-A-98/26074
- WILLS M R ET AL: "THE HUMAN CYTOTOXIC T-LYMPHOCYTE (CTL) RESPONSE TO CYTOMEGALOVIRUS IS DOMINATED BY STRUCTURAL PROTEIN PP65: FREQUENCY, SPECIFICITY, AND T-CELL RECEPTOR USAGE OF PP65-SPECIFIC CTL" JOURNAL OF VIROLOGY,US,THE AMERICAN SOCIETY FOR MICROBIOLOGY, Bd. 70, Nr. 11, 1. November 1996 (1996-11-01), Seiten 7569-7579, XP002060343 ISSN: 0022-538X
- ENDRESZ V ET AL: "Induction of human cytomegalovirus (HCMV)-glycoprotein B (gB)-specific neutralizing antibody and phosphoprotein 65 (pp65)-specific cytotoxic T lymphocyte responses by naked DNA immunization" VACCINE,GB,BUTTERWORTH SCIENTIFIC. GUILDFORD, Bd. 17, Nr. 1, 1999, Seiten 50-58, XP004139032 ISSN: 0264-410X

## Beschreibung

Die vorliegende Erfindung betrifft virale Partikel, die nach HCMV-Infektion von Säugerzellen freigesetzt werden, sowie ihre Verwendung als Impfstoff.

Das humane Cytomegalovirus (HCMV), ein β-Herpesvirus ist ein ubiquitär vorkommender Krankheitserreger. Die Infektion mit HCMV verläuft beim Immungesunden üblicherweise unbemerkt mit allenfalls leichten und unspezifischen Symptomen. Demgegenüber kommt es bei bestimmten Risikogruppen, beispielsweise bei immunsupprimierten Patienten wie AIDS-Patienten oder Transplantat-Empfängern, sowie nach pränataler Infektion zu schwerwiegenden Manifestationen der HCMV-Infektion.

Zur Behandlung der HCMV-Infektion stehen Chemotherapeutika zur Verfügung. Begrenzt wird der Erfolg der antiviralen Chemotherapie der HCMV-Infektion jedoch vor allem durch die Toxizität der Medikamente und die Entwicklung resistenter Virusvarianten bei längerer Behandlungsdauer. Auch hat sich der prophylaktische oder therapeutische Einsatz von antiviralen Hyperimmunseren als nur begrenzt wirksam erwiesen.

Bereits seit vielen Jahren wird an der Entwicklung einer Vakzine gegen HCMV gearbeitet. So wurde versucht, mit abgeschwächten (attenuierten) Lebendimpfstoffen die gewünschte Immunität zu induzieren. Allerdings erwies sich dieser Impfstoff als nur begrenzt wirksam. Gründe hierfür können unter anderem die eingeschränkte Vermehrungsfähigkeit derartiger attenuierter Viren im Menschen und stammspezifische Varianzen in der Antigenität sein. Neben diesen Unzulänglichkeiten bei der Induktion der bleibenden Immunität muß die Verwendung einer Lebend-Vakzine kritisch betrachtet werden; fehlende Erkenntnisse über die pathogenetischen Mechanismen bei der HCMV - Infektion und das Risiko der Reaktivierung des Impfstammes nach Immunsuppression lassen die Verwendung eines Lebend-Impfstoffes in dieser klinischen Situation zumindest fragwürdig erscheinen.

Um diese Risiken zu umgehen, werden in letzter Zeit Strategien zur Entwicklung von Spaltvakzinen gegen HCMV bevorzugt verfolgt, die Proteine aus der viralen Hülle, synthetisiert in unterschiedlichen Expressionssystemen, enthalten. Derartige Hüllproteine, vor allem die Glykoproteine gB und gH sind die wesentlichen Zielantigene neutralisierender Antikörper gegen HCMV. Neutralisierende Antikörper sind in der Lage, die Infektion zu verhindern. Sowohl im Versuchstier als auch in klinischen Studien war es möglich, solche neutralisierenden Antikörper mit einer gB-Spaltvakzine zu induzieren. Beim Menschen erwies sich die so induzierte Antikörperantwort jedoch als kurzlebig und nicht in allen Fällen geeignet, die Infektion zu verhindern. Dies steht der breiten Anwendung von Spaltvakzinen, die ausschließlich auf gB des HCMV beruhen, entgegen. Als Gründe für die begrenzte Wirksamkeit derartiger Antigenzubereitungen werden wiederum stammspezifische Varianzen in der Immunantwort, fehlende Induktion einer hinreichenden zellulären Immunantwort sowie strukturelle Restriktionen des verwendeten Antigens, dessen Epitope z.T. als konformations-abhängig bekannt sind, diskutiert.

Die WO 98/26074 beschreibt ein Fusionsprotein mit dem T-Zellenantigen pp65 als Impfstoff. Endresz et al. (Vaccine, 1999, S. 50 - 58) offenbaren Antikörper gegen das HCMV Glycoprotein gB. In beiden Fällen liegt das Impfstoffmaterial jedoch nicht in der Form viraler Partikel vor.

Aufgrund dieser Erfahrungen müssen daher an einen wirksamen und breit einsetzbaren Impfstoff gegen HCMV folgende Anforderungen gestellt werden:
(1) Langanhaltende Induktion neutralisierender Antikörper, die in Stamm-übergreifender Weise vor der Infektion mit HCMV schützen. Hierzu erforderlich ist die effiziente Induktion einer sog. "Helferzell-Antwort" (CD4-positive T-Lymphozyten) gegen HCMV zur Unterstützung der Reifung von Antikörper-sezernierenden B-Lymphozyten.
(2) Induktion der Bildung von zytotoxischen T-Zellen gegen HCMV. Derartige Lymphozyten sind von entscheidender Bedeutung, um eine stattgehabte Infektion mit HCMV zu terminieren und die Ausbreitung des Virus im Körper zu begrenzen.
(3) Minimierung der Nebenwirkungen durch die Vakzine. Das Risiko, welches durch ein verimpftes, vermehrungsfähiges Virus, das nach derzeitiger Kenntnis die Fähigkeit zur Etablierung von Latenz hätte, nach Immunsuppression ausgehen könnte ist nicht abschätzbar. Ziel sollte daher sein, nicht-vermehrungsfähiges virales Antigen als Impfstoff zuzubereiten.

Um die genannten Bedingungen für eine HCMV - Vakzine zu erfüllen, muß der Impfstoff die relevanten Antigene für die Induktion neutralisierender Antikörper und zur Stimulation von Helferzellen (T_{H}-Lymphozyten) sowie von zytotoxischen T-Zellen (CTL) enthalten.

Neutralisierende Antikörper werden nach HCMV-Infektion nach derzeitigem Kenntnisstand ausschließlich gegen virale Hüllproteine, und hier v.a. gegen die Glykoproteine gB und gH gebildet.

T_{H}-Zellen werden vorwiegend gegen Tegumentproteine des Virus, und hier besonders gegen das sog. pp65 (ppUL83) gebildet. Das pp65 ist darüber hinaus ein wesentliches Antigen zur Induktion von CTL gegen HCMV. Das pp65 wird nicht nur wie üblich nach Neusynthese durch Zellen im Zusammenhang mit MHC-Klasse I Molekülen präsentiert ; es kann auch durch sog. "exogene Beladung" in den MHC-I Präsentationsweg eingeschleust werden.

Die benannten Antigene sind die wesentlichen Bestandteile von defekten viralen Partikeln, die während der Infektion von primären humanen Fibroblastenkulturen durch HCMV synthetisiert und ins Kulturmedium freigesetzt werden. Bei diesen sogenannten "Dense Bodies" (DB) handelt es sich um im Elektronenmikroskop sichtbare Strukturen, die zu über 90% ihrer Proteinmasse aus pp65 bestehen. Sie werden vergleichbar zu Viruspartikeln mit einer durch virale Glykoproteine modifizierten zellulären Lipidmembran versehen und aus der Zelle ausgeschleust. Die viralen Glykoproteine liegen mit hoher Wahrscheinlichkeit in dieser Hülle in natürlicher Konformation vor. Da DB keine virale DNA und kein virales Capsid enthalten, sind sie nichtinfektiös. Sie können nach etablierten Methoden aus dem Zellkulturüberstand in großer Menge angereichert werden.

Eine Aufgabe der Erfindung bestand darin, einen wirksamen und breit einsetzbaren Impfstoff gegen HCMV bereitzustellen.

Die vorliegende Erfindung beschreibt virale Partikel, die nach Infektion von Säugerzellen durch humanes Cytomegalovirus (HCMV) freigesetzt werden. Diese Partikel können als präventiver oder therapeutischer Impfstoff gegen Infektionen durch das HCMV eingesetzt werden.

Die erfindungsgemäßen Partikel sind von einer Lipidmembran umschlossen, die es ermöglicht, daß die Partikel mit bestimmten Säugerzellen fusionieren, so daß ihr Inhalt ins Cytoplasma der Zellen gelangt. Die Membran der Partikel enthält virale Glykoproteine, die die Hauptantigene für virusneutralisierende Antikörper darstellen. Die Partikel sind auch dadurch gekennzeichnet, daß sie keine virale DNA und keine Capside enthalten. Weiterhin enthalten sie das virale T-Zellantigen pp65 (ppUL83), welches sowohl die Bildung von T-Helferzellen anregt, als auch ein wesentliches Antigen zur Induktion von cytotoxischen T-Lymphozyten (CTL) gegen HCMV ist.

Durch diese Eigenschaften, vor allem die Kombination von Antigenen, die sowohl neutralisierende Antikörper als auch eine ausreichende zelluläre Antwort induzieren können, eignen sich die Partikel als Impfstoffe gegen HCMV.

"Dense Bodies" wurden in der Literatur bereits beschrieben (Gibson et al. Birth Defects: Original Article Series 20, 1 (1984) 305-324; Virology 66 (1975) 464-473). In diesem Zusammenhang wurde ihre mögliche Verwendung als Impfstoff diskutiert. Jedoch wurden keine Versuche gezeigt, die belegen, daß "Dense Bodies" tatsächlich die erhofften Wirkungen zeigen. Außerdem ist gezeigt worden, daß "Dense Bodies" antigene Wirkung besitzen (Jahn et al., J. gen. Virol. 68 (1987) 1327-1337). Diese Versuche charakterisieren jedoch nicht die induzierte Immunantwort, weshalb keine Information über die Eignung als Impfstoff abgeleitet werden kann.

Daß die erfindungsgemäßen Partikel eine hohe Antigenität aufweisen und die Bildung neutralisierender Antikörper induzieren können, ist in den Beispielen 1 und 2 gezeigt. Die Induktion von Virus-neutralisierenden Antikörpern hält lange an (Beispiel 3), was eine weitere Voraussetzung für einen erfolgreichen Impfstoff ist. Die in den Beispielen 1-3 erzielte Immunantwort ist um so erstaunlicher als die Immunisierung ohne die Verwendung von Adjuvans durchgeführt wurde. Nebenwirkungen von Vakzinen, die mit Adjuvans verabreicht werden, entfallen somit. Es werden durch die erfindungsgemäßen Partikel auch cytotoxische T-Lymphozyten (CTL) aktiviert (Beispiele 4 und 5).

Schließlich induzieren DB unabhängig vom Applikationsweg T-Helferzell Antworten vom Th1-Typ (Beispiel 6).

Diese Beispiele belegen, daß die erfindungsgemäßen Partikel als Impfstoff gegen HCMV geeignet sind.

In einer weiteren Ausführungsform werden Partikel beschrieben, die ein Fusionsprotein enthalten, das zu einem Teil einen oder mehrere Abschnitte des viralen T-Zell-Antigens pp65 (ppUL83) bzw. das vollständige Protein umfaßt und zu einem weiteren Teil einen oder mehrere Abschnitte eines oder mehrerer anderer Proteine. Dadurch kann die Antigenität der Partikel optimiert werden, da dieses Fusionsprotein in großer Menge in den Partikeln enthalten ist. Daneben ist bekannt, daß die Expression von Antigenen der zellulären und humoralen Immunantwort in einem Molekül die Antigenität deutlich steigern kann. Die verschiedenen Abschnitte von pp65 und der anderen Proteine können direkt miteinander fusioniert sein, es können sich zwischen den verschiedenen Abschnitten aber beispielsweise auch "Linker"-Sequenzen befinden, die nicht natürlicher Bestandteil eines der beteiligten Proteine sind. Derartige Sequenzen können sich aufgrund der Klonierung ergeben oder bewußt eingeführt werden, um die Eigenschaften des Antigens zu beeinflussen. Vorzugsweise enthält das Fusionsprotein aber keine Fremdsequenzen, die nicht Bestandteil eines der Fusionspartner sind. In solchen Ausführungsformen besteht das Fusionsprotein aus einem oder mehreren Teilen von pp65 und einem oder mehreren Teilen eines oder mehrerer anderer Proteine.

Für alle im folgenden angeführten Ausführungsformen gilt, daß das gesamte pp65 oder ein oder mehrere Teile davon, in dem Fusionsprotein enthalten sein können. Die Angabe "ein Fusionsprotein (bestehend) aus pp65" ist im Sinne dieser Anmeldung nicht als auf gesamtes pp65 beschränkt zu verstehen. Ein in dem Fusionsprotein enthaltener "Teil" oder "Abschnitt" eines Proteins umfaßt wenigstens 6, bevorzugt wenigstens 8, am bevorzugtesten wenigstens 9, 15 oder 20 aufeinanderfolgende Aminosäuren des Proteins, von dem er herstammt.

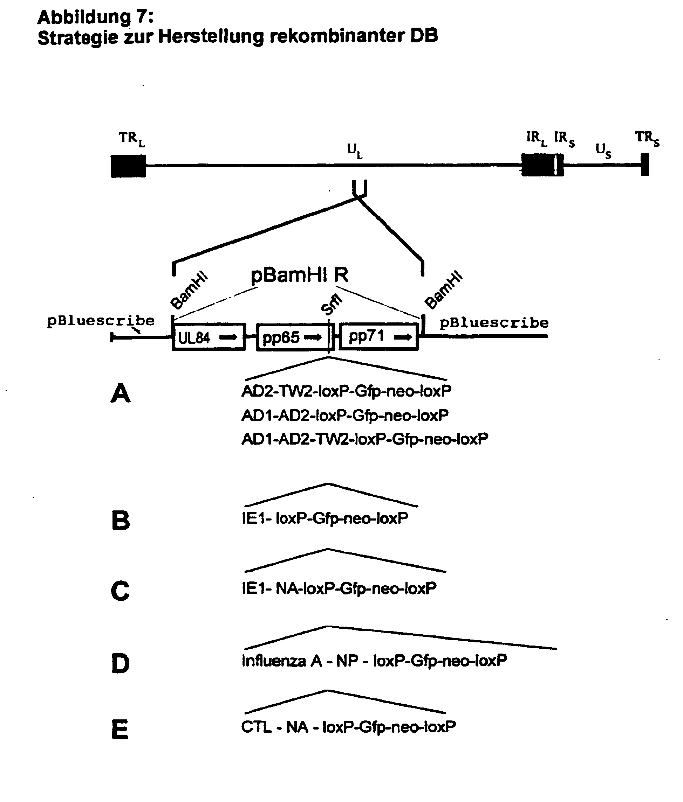

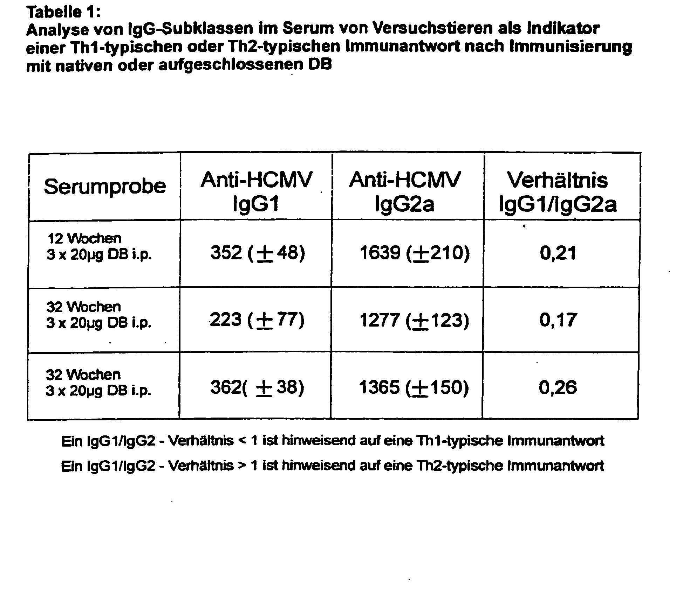

Eine bevorzugte Ausführungsform enthält ein Fusionsprotein aus pp65 (ppUL83) und einem oder mehreren neutralisierenden Epitopen der viralen Glykoproteine gB oder gH. Derartige Partikel können wie in Abb. 7A dargestellt generiert werden. Über antigenspezifische Aufnahme kann das Fusionsprotein in Glykoprotein-spezifische B-Zellen gelangen, welche wiederum die Epitope sowohl der Glykoproteine als auch des pp65 im Kontext von MHC-Klasse II präsentieren können. Daneben können Anteile des Fusionsproteins auch durch professionelle antigenpräsentierende Zellen (APC) im Kontext von MHC-Klasse II präsentiert werden. In beiden Fällen kommt es zur effizienten Stimulation der T_{H}-Antwort sowohl gegen pp65 als auch gegen virale Glykoproteine. Diese T_{H}-Zellen sind in der Lage, Glykoprotein-spezifische B-Zellen, die Peptide des pp65 und viraler Glykoproteine im Kontext von MHC-Klasse II präsentieren, sowohl homolog als auch heterolog zur Bildung neutralisierender Antikörper zu stimulieren. Daneben können derartige Partikel ähnlich wie infektiöse Virionen in Zellen aufgenommen und Peptide des pp65 durch exogene Beladung in den MHC-Klasse I Weg eingeschleust werden. Dadurch wird, für Totvakzinen ungewöhnlich, eine Stimulation der CTL-Antwort gegen HCMV erreicht.

In einer weiteren bevorzugten Ausführungsform enthalten die Partikel ein Fusionsprotein bestehend aus pp65 und einem oder mehreren Teilen eines weiteren Proteins von HCMV, dem IE1-Protein (ppUL123). Insbesondere sollen solche Teile des IE1-Proteins enthalten sein, gegen die während der natürlichen Infektion im Menschen zytotoxische T-Zellen gebildet werden. Peptide des IE1-Proteins werden z.T. durch andere MHC-Klasse I Moleküle präsentiert als Peptide des pp65. Durch Hinzunahme solcher weiterer "CTL-Epitope" aus IE1 soll gewährleistet werden, daß nach Immunisierung Impflinge, die unterschiedliche MHC-Klasse I Moleküle exprimieren, in möglichst umfassender Weise CTL gegen HCMV generieren können.

In einer weiteren bevorzugten Ausführungsform enthalten die Partikel ein Fusionsprotein bestehend aus pp65, aus einem oder mehreren neutralisierenden Epitopen der HCMV-Glykoproteine und aus einem oder mehreren CTL-Epitopen aus IE1. Durch Fusion von pp65 mit neutralisierenden Epitopen und CTL-Epitopen soll gewährleistet werden, daß gleichzeitig sowohl neutralisierende Antikörper als auch CTL von Impflingen in möglichst umfassender Weise, d.h. von möglichst vielen Personen mit unterschiedlichem MHC-Klasse I Muster gebildet werden können.

In einer weiteren bevorzugten Ausführungsform enthalten die Partikel ein Fusionsprotein aus pp65 und einem oder mehreren Epitopen eines anderen humanpathogenen Erregers. Als Anteile anderer humanpathogener Erreger kommen Antigene in Frage, gegen die im Menschen neutralisierende Antikörper gebildet werden. Durch Fusion derartiger "neutralisierender Antigene" mit dem T-Zellantigen pp65 kann eine deutliche Steigerung der Immunantwort (Antikörperantwort) im Vergleich zur Verwendung des isolierten "neutralisierenden Antigens" erwartet werden. Als Beispiele derartiger "neutralisierender Antigene" sind Oberflächenproteine des Hepatitis-B-Virus (aus dem Bereich HBsAG), des Hepatitis C-Virus (z.B. E2), der humanen Immundefizienzviren (HIV, aus dem Bereich Env), des Influenza-Virus (Hämagglutinin, Neuraminidase, Nukleoprotein) oder anderer viraler Erreger zu nennen. Als weitere humanpathogene Erreger kommen Bakterien, wie Hämophilus influenzae, Bordetella pertussis, Mykobacterium tuberculosis, Neisseria meningitidis und andere in Frage. Schließlich könnten Antigene von eukaryonten Erregern wie Plasmodien (Malaria) mit pp65 fusioniert werden.

In einer weiteren bevorzugten Ausführungsform enthalten die Partikel ein Fusionsprotein bestehend aus pp65 und einem oder mehreren Anteilen von Proteinen anderer Erreger, gegen die bei natürlicher Infektion mit diesen Erregern im Menschen CTL generiert werden. Als Beispiele derartiger CTL-Epitope können Anteile von Proteinen des HIV-1, des HBV, des HCV oder von Influenza-Virus genannt werden. Durch ein derartiges Vorgehen sollen die einzigartigen immunogenen Eigenschaften von DB dazu genutzt werden, um protektive CTL gegen heterologe Erreger im Menschen zu generieren.

In einer weiteren bevorzugten Ausführungsform enthalten die Partikel ein Fusionsprotein bestehend aus pp65, aus einem oder mehreren neutralisierenden Epitopen eines heterologen Erregers und aus einem oder mehreren CTL-Epitopen des gleichen Erregers. Durch diese Fusion soll gewährleistet werden, daß Impflinge sowohl protektive Antikörper als auch CTL gegen diesen Erreger bilden können.

Die Erfindung betrifft außerdem virale Partikel, die mindestens 2 verschiedene Glykoproteine enthalten, die Varianten desselben Glykoproteins von verschiedenen Stämmen des HCMV sind.

Eine bevorzugte Ausführungsform enthält genau 2 Varianten, wobei die eine der Variante des HCMV-Stammes Towne, die andere der Variante des HCMV-Stammes Ad169 entspricht. Die bevorzugte Ausführungsform enthält das Glykoprotein gB sowohl des Stammes Towne als auch das des Stammes Ad169.

Diese beiden Proteine können mit gleicher Effizienz in die Membran von rekombinanten "Dense Bodies" in der infizierten Zelle eingebaut werden. Derartige rekombinante "Dense Bodies" sind geeignet, neben der Stamm-übergreifenden auch die Stammspezifische neutralisierende Immunantwort gegen die beiden Prototypstämme von HCMV zu induzieren.

Schließlich ist ein weiterer Gegenstand der Erfindung ein Verfahren, wonach virale Partikel bereitgestellt werden, die völlig frei von infektiösen Viruspartikeln sind. Wenn Partikel aus einer Zellpopulation hergestellt werden, die mit HCMV infiziert worden ist, besteht ein Risiko, daß infektiöse Viruspartikel bei der Reinigung der Partikel mit verschleppt werden. Dies stellt einen Nachteil für einen Impfstoff dar.

Das erfindungsgemäße Verfahren minimiert dieses Risiko. Dazu wird zunächst ein HCMV-Stamm hergestellt, der eine Deletion in einem essentiellen Gen trägt. Darunter ist eine Deletion der Funktion des Gens zu verstehen. In den meisten Fällen beruht dies auf der Abwesenheit eines funktionellen Genprodukts, es kann aber auch die Funktion einer regulatorischen Gensequenz derart gestört sein, daß das HCMV nicht mehr vermehrungsfähig ist. Dies kann durch Veränderung der Nukleinsäuresequenz des HCMV geschehen, zum Beispiel durch Punktmutationen, tatsächliche Deletionen, Insertionen oder sonstige Mutationen. Dieses defekte Virus kann nur in Zellen vermehrt werden, die das Gen, das im HCMV deletiert worden ist, exprimieren und somit für den Zusammenbau der Virionen zur Verfügung stellen. Primäre Fibroblasten stellen derzeit das einzige vernünftig permissive System für die in vitro-Vermehrung des HCMV dar. Bislang war es nur mit Hilfe von retroviralen Transfer-Methoden möglich, solche Zellen stabil zu transfizieren. Das ist jedoch ein gravierender Nachteil, wenn mit Hilfe solcher Zellen Impfstoffe hergestellt werden sollen. Das erfindungsgemäße Verfahren stellt stabil transfizierte Zellen zur Verfügung, die ohne retroviralen Gentransfer hergestellt werden können, in denen aber auch HCMV vermehrt werden kann.

Eine bevorzugte Ausführungsform sind humane Vorhautfibroblasten, die mit dem Hauptcapsidprotein-Gen UL86 stabil transfiziert worden sind. Bevorzugt wird die Transfektion mit einem lipidhaltigen Hilfsmittel durchgeführt, was zu einer sehr hohen Transfektionseffizienz führt. Bei einer bevorzugten Ausführungsform wird für die Transfektion das "Fugene-Reagenz" eingesetzt, das von Roche Diagnostics, Mannheim käuflich erhältlich ist. Diese Ausführungsform ist in Beispiel 7 beschrieben.

In diesen Zellen kann defizientes Virus vermehrt werden, dessen Hauptcapsidprotein-Gen UL86 deletiert wurde. Infiziert man "nicht-komplementierende" Fibroblasten mit diesem defizienten Virus, dann können daraus virale Impfstoffpartikel isoliert werden, die frei von infektiösen Viruspartikeln sind.

Eine weitere Möglichkeit, die erfindungsgemäßen Partikel ohne infektiöses Risiko herzustellen, ist die Rekonstitution der Partikel in Zellen, ohne daß mit HCMV infiziert wird. Dazu müssen in diesen Zellen alle Gene exprimiert werden, die für Bestandteile der Partikel codieren. Diese Gene müssen dazu in die Zellen eingeschleust werden.

Vorzugsweise werden dazu Insektenzellen benutzt, die von Baculoviren infiziert werden. Die Gene, die für die Polypeptidbestandteile der Partikel codieren, werden in Baculovirusexpressionsvektoren kloniert. Nach Herstellung von rekombinanten Baculoviren werden Insektenzellen, vorzugsweise Sf9-Zellen, durch die verschiedenen Viren koinfiziert. Die Gene werden in den Insektenzellen exprimiert, und die resultierenden Polypeptide lagern sich zu den gewünschten Partikeln zusammen. Schließlich kommt es zu einer Freisetzung der Partikel durch die Insektenzellen. Dies stellt eine Möglichkeit dar, nichtinfektiöse Partikel herzustellen, die als Impfstoff verwendet werden können.

Alternativ können die Bestandteile, die notwendig für die Rekonstitution von DB sind, in rekombinante Baculoviren unter Kontrolle des HCMV-Major IE-Promotor-Enhancers (MIEP) kloniert werden. Es ist gezeigt, daß rekombinante Baculoviren höhere eukaryonte Zellen wie beispielsweise Säugerzellen infizieren können und daß Fremdgene unter der Kontrolle eines starken eukaryonten Promotors wie des MIEP in solchen Zellen stark exprimiert werden. Vorteil eines solchen Vorgehens wäre es, daß eventuell wichtige Modifikationen, wie Glykosylierung, von antigenen Proteinen der DB in Säugerzellen in natürlicherer Weise erfolgen könnten als in Insektenzellen. Daneben gibt es eine Reihe von solchen Zellinien, die bereits für die Impfstoff-Herstellung zugelassen sind.

Die beiliegenden Abbildungen werden im folgenden erläutert:

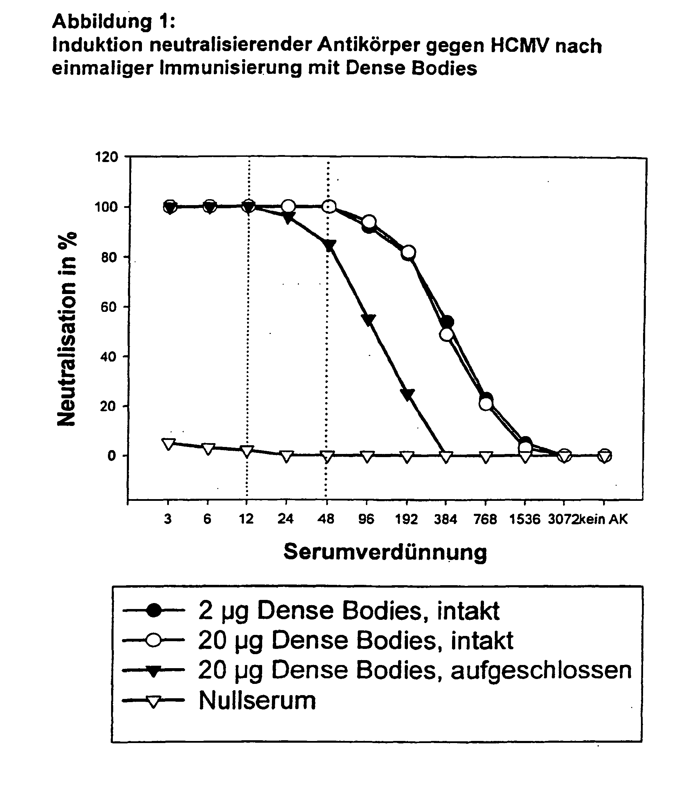

**Abbildung 1** zeigt das Ergebnis des Neutralisationstestes zum Nachweis HCMV-neutralisierender Antikörper in Seren von Mäusen nach einmaliger Immunisierung mit "Dense Bodies"-Präparationen gemäß Beispiel 1. Als 100% Neutralisation wird gewertet, wenn im Vierfach-Ansatz in der unter Beispiel 1 beschriebenen Färbemethode kein positiver Zellkern mehr nachweisbar ist (gestrichelte Linien). Der Grad der Neutralisation bei einer bestimmten Serumverdünnung ergibt sich aus der Zahl positiver Zellen im Vierfachansatz bezogen auf die Zahl der positiven Zellen im Ansatz ohne Antikörper.

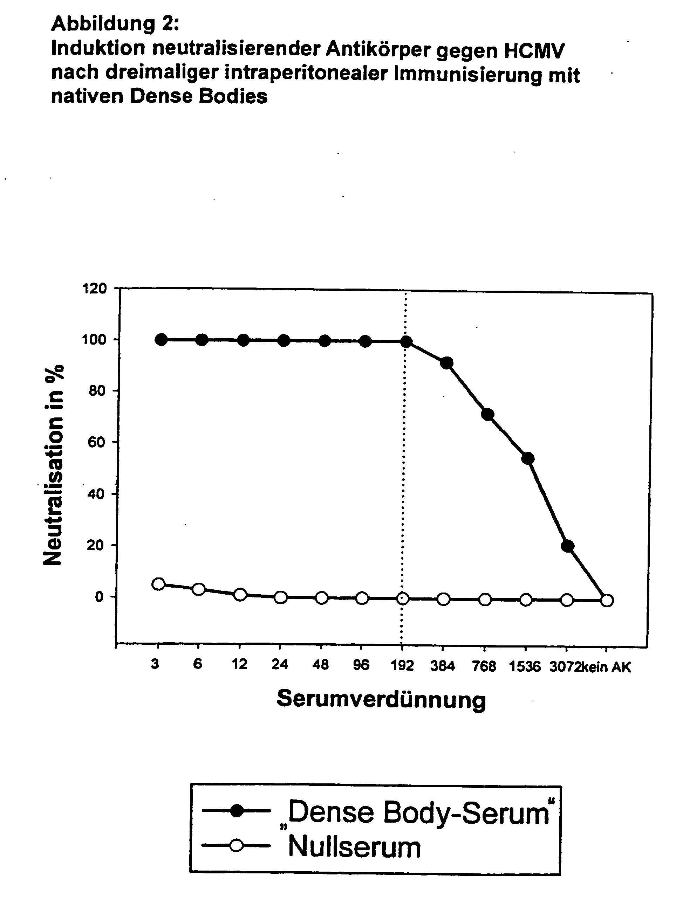

**Abbildung 2** zeigt das Ergebnis des Neutralisationstestes zum Nachweis HCMV-neutralisierender Antikörper in Seren von Mäusen nach dreimaliger intraperitonealer Immunisierung im Abstand von jeweils 4 Wochen mit jeweils 20µg nativen "Dense Bodies", wie in Beispiel 2 beschrieben. Die Auswertung des Testes erfolgte wie in Abb. 1 beschrieben. Die gepunktete Linie gibt den Serum-Titer an, bei dem 100%ige Neutralisation vorlag. Als "Nullserum" wurde das Serum einer Maus eingesetzt, die nicht mit HCMV - Partikeln immunisiert worden war.

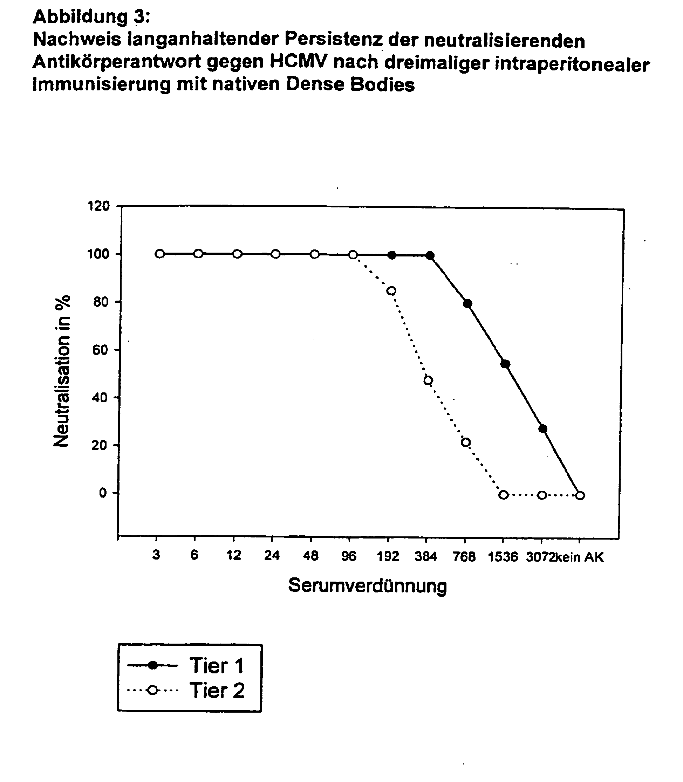

**Abbildung 3** zeigt das Ergebnis eines Neutralisationstestes zum Nachweis einer langanhaltenden Produktion neutralisierender Antikörper nach dreimaliger intraperitonealer Immunisierung (Abstand 4 Wochen) mit jeweils 20µg nativen "Dense Bodies" gemäß Beispiel 3. Die Auswertung erfolgte wie in Abb. 1 beschrieben.

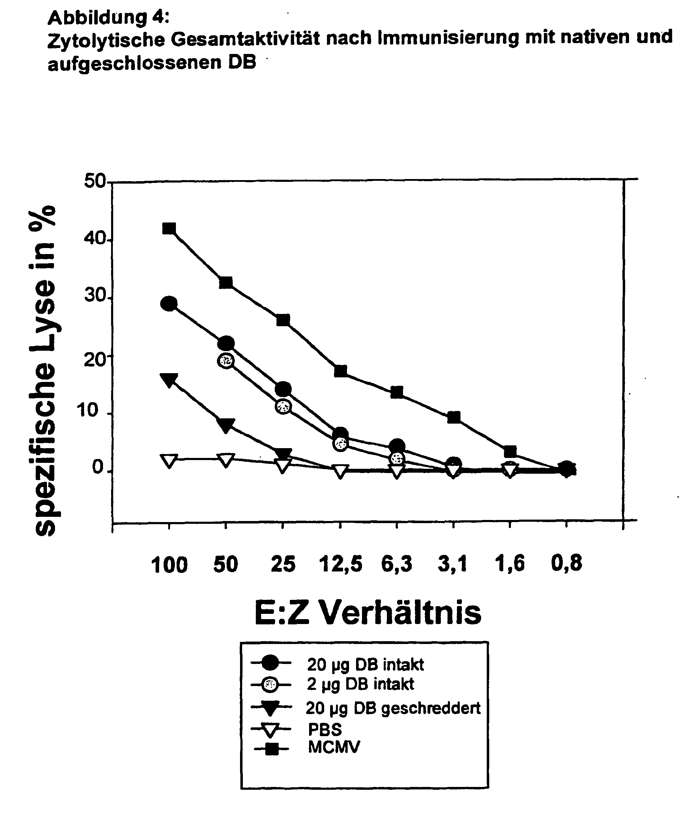

**Abbildung 4** zeigt das Ergebnis einer Analyse der cytolytischen Gesamtaktivität nach Immunisierung von Mäusen mit nativen und aufgeschlossenen "Dense Bodies" mit Hilfe der anti-CD3ε vermittelten redirigierten Lyse, wie in Beispiel 4 beschrieben. Aufgetragen ist die jeweilig beobachtete lytische Aktivität gegen das Verhältnis von Effektorzellen zu Zielzellen.

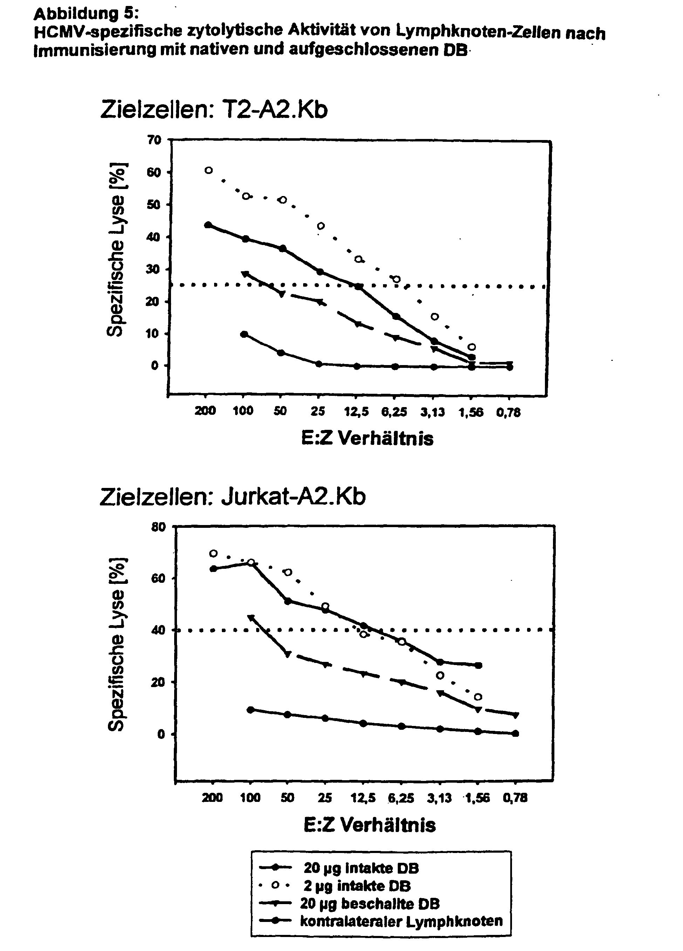

**Abbildung 5** zeigt das Ergebnis einer Analyse der HCMV-spezifischen zytolytischen Aktivität von Lymphknoten-Zellen nach Immunisierung mit nativen und aufgeschlossenen DB, wie in Beispiel 5 beschrieben. Aufgetragen ist die jeweilig beobachtete lytische Aktivität, gemessen anhand des Ausmaßes der Freisetzung von radioaktiv-markiertem Chrom, gegen das eingesetzte Verhältnis von Effektorzellen zu Zielzellen (E:Z Verhältnis). Die gepunkteten Linien markieren beispielhaft die relative Zahl von Effektorzellen, welche notwendig war, um einen vorgegebenen Lysewert zu erzielen. Es ist ersichtlich, daß deutlich niedrigere Zahlen an Zellen von Tieren, welche mit unbehandelten DB immunisiert worden waren eingesetzt werden mußten, um den gleichen Lyse-Wert zu erreichen.

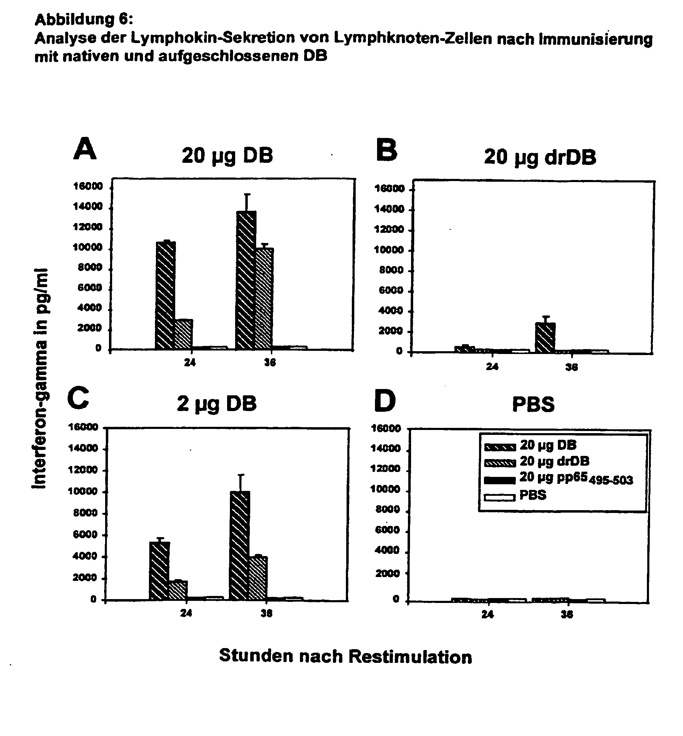

**Abbildung 6** zeigt das Ergebnis einer Analyse der Art der Th-Immunantwort nach Immunisierung mit nativen und aufgeschlossenen DB anhand der Lymphokin-Freisetzung durch Lymphknoten-Zellen, wie in Beispiel 6 beschrieben. Aufgetragen ist die Menge an freigesetztem IFN-γ gegen die Zeitdauer nach Restimulation. A) Immunisierung mit 20µgDB, B) Immunisierung mit 20µg aufgeschlossenen DB (drDB), C) Immunisierung mit 2µgDB, D) Immunisierung mit PBS (negative Kontrolle). Die zur Restimulation eingesetzten Antigene sind in der Einblendung von D) gezeigt. IL-5 Sekretion wurde parallel bestimmt. In keinem Fall konnte eine signifikante Sekretion von IL-5 nachgewiesen werden.

**Abbildung 7** zeigt die Strategie zur Generierung von rekombinanten DB, die Fusionsprotein mit homologen oder heterologen Antigenen enthalten und die in Beispiel 8 beschrieben ist.

**Tabelle 1** zeigt das IgG-Subklassenprofil HCMV-spezifischer Antikörper im Serum von Balb/cJ Mäusen, die in vierwöchigen Intervallen mit jeweils 20µgDB intraperitoneal immunisiert worden waren. Die Seren wurden 12 Wochen bzw. 32 Wochen nach erster Injektion gewonnen und wie in Beispiel 6 beschrieben analysiert. Angegeben ist diejenige Endpunkt-Verdünnung eines Testserums, bei der die Absorption im ELISA der 2,5 fachen Absorption eines Nullserums entspricht. Aus diesen Werten wurde jeweils das Verhältnis IgGl/IgG2a bestimmt.

Die Erfindung wird in den nachfolgenden Beispielen weiter erläutert.

### Beispiel 1:

### Induktion von neutralisierenden Antikörpern durch native versus aufgeschlossene DB nach einmaliger Immunisierung der Maus in die Pfote

Je 2 BALB/cJ Mäuse (weiblich, 9-10 Wochen alt) wurden entweder mit 2 µg oder mit 20 µg gereinigten DB oder mit 20 µg aufgeschlossenen DB durch Injektion in die Hinterpfote immunisiert. Die Blutentnahme erfolgte am Tag 55 nach Immunisierung durch Herzpunktion. Serum wurde durch Abtrennung der zellulären Fraktion nach Agglutination gewonnen.

Die Mäuseseren wurden hinsichtlich ihrer Kapazität zur Neutralisation des HCMV im sog. "Neutralisationstest" untersucht. Die Untersuchung wurde nach folgendem Protokoll durchgeführt:

Die Seren und die Kontrollantikörper 14-4b (neutralisierend) und B1B6 (nicht neutralisierend) wurden zunächst 1:3 in Kulturmedium vorverdünnt und dann in einer 48-well-Platte zehnmal durch serielle Verdünnung jeweils 1:2 verdünnt:

Von jeder Verdünnung lagen 250 µl vor.

Zu jeder Verdünnung wurden 250 µl (gleiches Volumen) eines HCMV Virusstocks (Ad169 mit einer TCID₅₀ von 10^{6,3}/ml (1 : 200 vorverdünnt 100µl + 19.9ml) vom 16.12.97) zugegeben und vorsichtig gemischt. Zuvor wurde das Virus nach dem Auftauen 20 sec auf einem "Vortexgerät" gemischt.

Das Virus-Antikörper-Gemisch wurde 4h bei 37°C im Brutschrank inkubiert.
Nach 3,5 h wurden abtrypsinierte humane Vorhautfibroblasten (HFF) ausgezählt und auf 6 x 10⁵ Zellen/ml eingestellt (das entspricht 1,5 x 10⁴ Zellen /25 µl).
In einer 96-well Platte wurden je 25 µl Zellsuspensionen in je 4 Reihen pro zu testendem Antikörper oder Antiserum vorgelegt. Nach 4 Stunden Inkubationszeit wurden je 100 µl der entsprechenden Virus-Antikörper-Verdünnung zugegeben.
Anschließend wurde über Nacht bei 37°C im CO₂-Inkubator inkubiert.

Am nächsten Tag wurden die Kulturen nach Verwerfen des Kulturüberstandes mit 96% EtOH 15 min bei -20°C fixiert und mit PBS gewaschen. Anschließend wurde als erster Antikörper der murine monoklonale Antikörper p63-27; (je 50 µl pro well) zugegeben und 1h bei 37°C inkubiert.

Nach erneutem Waschen wurde der 2. Antikörper, ein mit Meerrettich-Peroxidase-gekoppeltes anti-Maus IgG (Dako, Hamburg, 1:500 in PBS) zugegeben und für 45 min bei 37°C inkubiert. Nach dieser Inkubation wurde erneut mit PBS gewaschen und anschließend eine AEC-Färbung zum Nachweis der spezifischen Antikörper-Reaktion durchgeführt.

Hierzu werden pro Vertiefung jeweils 100µl einer AEC-Substratlösung zugegeben und für 10-20 Min. bei 37°C im Dunkeln inkubiert. Die Reaktion wird nachfolgend mit PBS gestoppt und die Vertiefungen mehrfach mit PBS gespült. Anschließend werden im Durchlicht die gefärbten Zellkerne identifiziert und ausgezählt.

### Reagenzien:

| | |
|---|---|
| AEC-Stammlösung: | 400mgAEC/100ml Dimethyl-Formamid (Sigma A-5754) |
| Acetat-Puffer: | 6,8g Na-Azetat, 2,88 ml Eisessig, ad 1 L Aqua dest (pH 4,9) |
| AEC-Gebrauchslösung: | AEC-Stammlösung 1:20 in Acetat-Puffer verdünnen und 2 x filtrieren. Kurz vor der Färbereaktion 1/1000 30%iges H₂O₂ zugeben. |

### Beispiel 2:

### Induktion von neutralisierenden Antikörpern durch native DB nach dreimaliger intraperitonealer Immunisierung der Maus

Zur Überprüfung der Induktion von Antikörpern wurde ein weiteres Immunisierungsschema eingesetzt. Hierzu wurden 5 Tiere dreimal (Zeitpunkte 0, 4 Wochen, 9 Wochen) mit jeweils 20µg unbehandelten DB intraperitoneal immunisiert. Die Blutentnahme erfolgte bei drei der Tiere in der Woche 12, bei weiteren zwei Tieren in der Woche 32.

Die gewonnenen Mäuseseren wurden hinsichtlich ihrer Kapazität zur Neutralisation des HCMV im Neutralisationstest untersucht. Dabei wurde methodisch wie unter Beispiel 1 dargestellt vorgegangen. Die Ergebnisse sind unten dargestellt und zeigen, dass die dreimalige Applikation von 20µg unbehandelter DB zu einer signifikanten Steigerung der neutralisierenden Antikörperproduktion im Vergleich zur einmaligen Applikation in die Pfote führt. Der erhaltene Wert der Neutralisation entspricht einem Titer, der üblicherweise mit Humanseren von seropositiven Spendern erhalten wird.

### Beispiel 3

### Induktion einer langanhaltenden neutralisierenden Antikörperantwort durch native DB nach dreimaliger intraperitonealer Immunisierung der Maus

Balb/cJ Mäuse wurden nach dem unter Beispiel 2 genannten Schema dreimal mit nativen DB intraperitoneal immunisiert. 32 Wochen nach der ersten Immunisierung wurde das Serum der Tiere wie oben beschrieben gewonnen und im Neutralisationstest untersucht. Es zeigte sich, dass neutralisierende Antikörper in hoher Konzentration noch nach relativ langer Zeit nach initialer Immunisierung nachweisbar blieben. Dies deutet darauf hin, dass DB anders als bislang verwendete Totimpfstoffe des HCMV eine langanhaltende neutralisierende Antikörperantwort induzieren können.

### Beispiel 4

### Induktion zytolytischer Zellen durch native versus aufgeschlossene DB

Im Rahmen dieses experimentellen Ansatzes wurde getestet, inwieweit die Applikation von nativen und aufgeschlossenen DB geeignet ist, im Versuchstier zytolytische Effektorzellen zu aktivieren. Hierzu wurde die Methode der anti-CD3 - vermittelten redirigierten Lyse eingesetzt. Das experimentelle Vorgehen ist im folgenden dargestellt:

Dense Bodies wurden aus dem Zellkultur-Überstand von humanen Vorhaut-Fibroblasten, welche mit dem Laborstamm Ad169 des HCMV infiziert worden waren zum Zeitpunkt des vollen zytopathogenen Effektes (6-8 Tage nach Infektion) aufgereinigt. Hierzu wurde das Kulturmedium gewonnen und virale Partikel durch Zentrifugation in einem SW28 Rotor bei 24.000 U/min (10°C) für eine Stunde sedimentiert. Das Sediment wurde anschließend in PBS resuspendiert und auf einen Glycerol-Tartrat Gradienten geschichtet. Nach Ultrazentrifugation wurden die den Dense Bodies zuzuordnenden, im Durchlicht sichtbaren Banden mit Hilfe einer Kanüle und einer lml Spritze abgezogen. Ein Aliquot wurde im SDS-PAGE hinsichtlich seiner Reinheit überprüft und die Menge an DB in der Präparation anhand der Färbeintensität gegen einen BSA-Standard mit Hilfe der Tina-Software der Fa. Raytest, Deutschland quantifiziert.
Zur Analyse, inwieweit die Hülle von DB eine Rolle für ihre Immunogenität spielt, wurde ein Teil der Präparationen in einer Weise behandelt, daß die Hülle von der Tegument-Kernstruktur abgetrennt wurde, die partikuläre Natur der Partikel aber erhalten blieb. Hierzu wurden dieser Anteil der DB 10 Zyklen von Einfrieren in flüssigem Stickstoff mit anschließendem Auftauen unterworfen. Danach wurden die Partikel für 10 Minuten in einem Sonifizier-Gerät der Fa. Branson (Sonifier 250; Einstellungen: Duty Cycle 10%, Stufe 3 - output control 2,5) beschallt. Die Struktur der so behandelten Partikel wurde im Elektronen-Mikroskop visualisiert, ihre physikalischen Eigenschaften im Sucrose-Gradienten analysisert.

Als Versuchstiere wurden weibliche, 9-10 Wochen alte BALB/cJ-Mäuse eingesetzt. Die Immunisierung der Mäuse erfolgte durch Applikation von je 50 µl Volumen, wobei das Material in PBS verdünnt wurde, in die rechte Hinterpfote. Es wurden 5 Tiergruppen immunisiert, jede Gruppe bestand aus 6 Tieren. Gruppe 1 wurde mit 2 x 10⁵ pfu gereinigtem murinem CMV (MCMV) in PBS immunisiert, Gruppe 2 mit je 20 µg intakten Dense Bodies (Menge im Coomassiegel auf den Gehalt von pp65 normiert), Gruppe 3 mit 2 µg intakten Dense Bodies, Gruppe 4 mit 20 µg mechanisch zerstörten Dense Bodies (siehe Protokoll), Gruppe 5 mit 50 µl PBS. Am achten Tag wurden die ipsi- sowie die kontralateralen poplitealen Lymphknoten präpariert. Die Lymphozyten wurden durch Zerreiben der Lymphknoten durch ein Metallsieb und mehrmaliges Waschen isoliert. Die Lymphknoten aus einer Gruppe wurden gepoolt. Nach Bestimmung der Zellzahl wurden die Lymphozyten in IL-2-haltigem Medium für acht Tage zwischenkultiviert (100 U rekombinantes humanes IL-2/ml T-Zell-Medium). Als T-Zellmedium wurde MEM alpha verwendet, das zusätzlich 10% FKS, 4 mM L-Glutamin, 100 U/ml Penicillin, 0,1 mg/ml Streptomycin, 10 µg/ml Kanamycin, 10 mM Hepes, 0,00035% β-Merkaptoethanol enthielt. Am 16. Tag wurde die redirigierte Lyse durchgeführt. Dazu wurden zunächst P815-Mastocytomazellen (ATCC Nummer: TIB-64; Organismus: Mus musculus D) mit Cr-51 markiert. Dazu wurden 1x10⁶ Zellen in 30 µl FKS resuspendiert und 75 Minuten mit 100 µCi Cr-51 (Natriumdichromat) bei 37°C markiert. Anschließend wurden die Zellen dreimal in P815 Medium gewaschen, um überschüssiges Chrom zu entfernen (P815-Medium: RPMI, 10 mM Hepes, 5% FKS, 2 mM L-Glutamin, 100 U/ml Penicillin, 0,1 mg/ml Streptomycin, 0,00035% β-Merkaptoethanol). Ein Teil der Zellpopulation wurde mit Anti-CD3 Antikörper beladen: Dazu wurden 5x10⁵ P815-Zellen 15 Minuten mit 5 µl Anti-CD3 Antikörper bei Raumtemperatur inkubiert (Hamster Anti-Mouse CD3ε; Southern Biotechnology Associates Inc. Birmingham, AL; 0,5 mg/ml in PBS). Anschließend wurden die Zellen zweimal gewaschen.

Der Zytotoxizitäts-Test wurde in 96-well Mikrotiterplatten durchgeführt:

Unterschiedliche Mengen Lymphozyten wurden in 100 µl ausgesät. Pro Well wurden je 1000 markierte Zielzellen (P815 bzw. P815/anti-CD3) in 100 µl zugegeben. Zur Bestimmung der maximalen Lyse wurden 1000 markierte Zielzellen direkt im γ-Counter gemessen. Zur Bestimmung der Spontanlyse wurden statt Effektorzellen 100 µl Medium zugegeben. Nach Zentrifugation (100 g, 4 Minuten) wurde 4 Stunden bei 37°C inkubiert. Anschließend wurde wiederum zentrifugiert (430 g, 4 Minuten) und 100 µl Überstand abgenommen, dessen Cr-51-Aktivität im γ-Counter gemessen wurde. Die Lysewerte für die jeweilige Effektorpopulation ergeben sich wie folgt: (gemessene Chromfreisetzung - Spontanfreisetzung) x100 / (Maximalfreisetzung - Spontanfreisetzung). Die spezifische redirigierte Lyse ergibt sich nach Subtraktion der Werte der für die kontralateralen Lymphknotenzellen bestimmten Lyse.

### Protokoll: mechanischer Aufschluß von "Dense Bodies".

Die mechanische Zerstörung der DB erfolgt durch zehnmaliges Einfrieren und Auftauen in flüssigem Stickstoff mit anschließender Sonifikation für 10 Minuten in einem Beschallungsgerät der Firma Branson (Sonifier 250; Einstellungen: Duty Cycle 10%, Stufe 3 - output control 2,5).

### Beispiel 5

### Induktion pp65-spezifischer CTL nach Immunisierung von HLA-A2.k^{b} transgenen Mäusen

Im Rahmen dieser Experimente wurde getestet, inwieweit die Applikation von nativen und aufgeschlossenen DB geeignet ist, im Versuchstier HCMV-spezifische zytolytische Effektorzellen zu aktivieren. Jeweils 8-10 Wochen alte Mäuse des Stammes C57/BL6, welche für das humane HLA-A2 Molekül transgen waren (C57/BL6-A2.k^{b}), wurden wie unter Beispiel 4 beschrieben mit 2µgDB, 20µgDB oder 20µg aufgeschlossenen DB, gelöst in PBS, ohne Zugabe von Adjuvans intraplantar immunisiert. Die Zellen der poplitealen Lymphknoten wurden wie beschrieben gewonnen, gezählt und in-vitro für 8 Tage unter Zugabe von 100 U/ml rekombinantem humanem Interleukin-2 in T-Zell-Medium kultiviert. Am 16. Tag wurde die HCMV-spezifische zytolytische Aktivität in diesen Zellpopulationen gemessen. Als Zielzellen für den Chromfreisetzungstest wurden humane T2-Zellen (ATCC CRL-1992) und Jurkat-Zellen (ATCC-Nummer TIB-152) eingesetzt, welche stabil das Gen für das chimäre MHC-Molekül A2.k^{b} exprimierten. Diese Zellen wurden wie in Beispiel 4 beschrieben mit Cr-51 markiert. Danach wurden je 5x10⁵ Zellen zum einen mit einem bekannten, von HLA-A2 präsentierten Peptid des pp65 beladen (Aminosäuren 495-503; Peptid synthetisch hergestellt von der Firma Jerini, Berlin), zum anderen mit einem irrelevanten, von HLA-A2 präsentierten Kontrollpeptid aus dem Tumorsuppressorprotein p53 (Aminosäuren 149-157) beladen. Dazu wurden die Peptide in einer Konzentration von 10⁻³M in PBS gelöst und anschließend in 10er Schritten weiterverdünnt. Je 5×10⁵ Zielzellen wurden dann in einem Volumen von 500 µl mit Peptiden in Endkonzentrationen von 10⁻⁵ bis 10⁻¹⁰M 1 Std bei 37°C inkubiert. Danach wurden die nicht-gebundenen Peptide durch Waschen in T-Zell-Medium entfernt. In Vortests wurden die optimalen Peptidkonzentrationen zur Beladung der beiden Zellinien mit 10⁻⁶ M für Jurkat-Zellen und 10⁻⁸ M für T2-Zellen bestimmt.

Chromfreisetzungstests wurden mit absteigendem Effektorzell:Zielzell (E:T) - Verhältnis durchgeführt (Abbildung 5). Dabei wurden die Werte der Chromfreisetzung, welche in einem parallelen Experiment nach Beladung der Zielzellen mit einem irrelevanten Peptid (durch HLA-A2 präsentiertes Peptid aus dem Tumorsuppressor Protein p53) erhalten wurden, von den Werten subtrahiert, die jeweils nach spezifischer Lyse von mit dem pp65 Peptid beladenen Zielzellen erhalten worden war. Es zeigte sich, daß die Immunisierung mit DB zur Generierung von pp65-spezifischen (HCMV-spezifischen) CTL geführt hatte. Dabei zeigten die Zellen der Tiere, welche mit 2µg DB immunisiert worden waren, etwas höhere Werte der Chromfreisetzung bei einem vorgegebenen E:T-Verhältnis als die Zellen der Tiere, die mit 20µg DB immunisiert worden waren. Die Zellen der Tiere, die mit aufgeschlossenen Partikeln immunisiert worden waren, zeigten zwar immer noch pp65-spezifische Zytolyse; die Chromfreisetzng als Maß für ihre zytolytische Effektoraktivität war jedoch bei gegebenem E:T-Verhältnis jeweils deutlich niedriger als die der Zellen von Tieren, die nicht-behandelte DB erhalten hatten. Insbesondere war bei vorgegebenem Chromfreisetzungs-Wert als Maß für die zytolytische Aktivität der Zellen, die aus mit aufgeschlossenen Partikeln behandelten Tieren erhalten wurden, die hierfür notwendige relative Anzahl an Effektorzellen (E:T-Verhältnis) deutlich höher. Diese Ergebnisse deuten zusammengefaßt darauf hin, daß (I) die Immunisierung von Versuchstieren mit dem "Totantigen" DB geeignet ist, HCMV-spezifische zytolytische T-Zellen zu generieren und daß (II) die Effizienz der zellulären Immunantwort von der zellulären Aufnahme der DB, vermutlich vermittelt durch eine intakte Hülle, abhängig ist. Dieses Ergebnis muß als ausgesprochen bemerkenswert bezeichnet werden, da eine effiziente Generierung von CTL gegen intrazelluläre Pathogene in der Regel von der Neusynthese der entsprechenden Antigene in der Zelle abhängt. Die durch DB vermittelte "exogene Beladung" des MHC-Klasse I Komplexes mit virusspezifischen Peptiden kann somit als außergewöhnlich effizient angesehen werden und sollte geeignet sein, protektive zytotoxische Effektorfunktionen gegen HCMV und gegen heterologe Erreger wie z.B. Influenza-Virus zu erzeugen.

### Beispiel 6:

### Immunisierung mit DB führt zur Induktion einer Th1-typischen T-Helfer-Zell Antwort

Die Induktion einer dauerhaften humoralen und zellulären Immunantwort hängt in entscheidender Weise davon ab, ob und in welcher Weise T-Helfer Lymphozyten induziert werden können. Insbesondere ist es bei Immunisierung gegen virale Erreger wünschenswert, eine Th1-typische Immunantwort zu erzeugen. Um zu prüfen, inwieweit die Immunisierung mit DB hierfür geeignet ist, wurden zwei verschiedene Versuchsansätze gewählt.

In einem ersten Versuchsansatz wurde geprüft, ob von Lymphknoten-Zellen von Versuchstieren, welche mit DB intraplantar immunisiert worden waren, nach Zwischenkultivierung Th1-typische (hier: Interferon-γ, IFN-γ) oder Th2-typische (hier Interleukin-5, IL-5) Lymphokine synthetisiert und freigesetzt werden. Es wurden Gruppen von je sechs Balb/cJ Mäusen mit 2µg DB, 20µg DB, 20µg aufgeschlossenen DB gelöst in PBS oder mit PBS alleine intraplantar immunisiert. Lymphknoten - Zellen wurden nach 8 Tagen nach Immunisierung wie in Beispiel 4 beschrieben gewonnen und in einer Dichte von 10⁶ Zellen/ml in RPMI 1640 Medium supplementiert mit 2mM L-Glutamin, 100U/ml Penicillin, 0,1mg/ml Streptomycin, 5% (v/v) FCS, 5x10⁻⁵M 2-Mercaptoethanol, 10mM Hepes ausgesät. Die Zellen wurden in Kultur mit entweder 20µg DB, 20µg aufgeschlossener DB, mit einem durch MHC-Klasse I (HLA-A2) präsentierten Peptid (pp65, 495-503) oder mit PBS restimuliert, indem diese Antigene dem Kulturmedium zugesetzt wurden. 24 und 36 Stunden nach Restimulation wurde der Zellkulturüberstand geerntet und 10 min bei 2500 Upm in einer Eppendorfzentrifuge abzentrifugiert um Zellreste abzutrennen. Mit Hilfe von kommerziell verfügbaren ELISA-Testkits (Endogen, Woburn, U.S.A.) wurde der Gehalt an IFN-γ und IL-5 im Überstand der Kulturzellen gemessen (Abbildung 6). Zur Standardisierung der gemessenen Werte wurde rekombinantes IFN-γ und IL-5, die in den gleichen Testkits mitgeliefert wurden, in dem verwendeten Zellkulturmedium gelöst und parallel mitanalysiert.

Die Lymphozyten von den Tieren, die mit 20µg oder 2µg DB immunisiert worden waren, sezernierten große Mengen an IFN-γ. Deutlich weniger IFN-γ wurde von Zellen von solchen Tieren sezerniert, welche mit aufgeschlossenen Partikeln immunisiert worden waren. IL-5 wurde in keinem Ansatz in signifikanter Menge gemessen. Diese Ergebnisse deuten darauf hin, daß die Immunisierung von Versuchstieren mit DB geeignet ist, eine Th1-typische Immunantwort zu induzieren und daß das Ausmaß der Induktion wiederum von der Integrität der DB-Membran abhängt.

Da bekannt ist, daß die Induktion einer Th1-typischen oder Th2-typischen Immunantwort unter anderem von der Applikationsform abhängt, wurden Mäuse in einem zweiten Versuchsansatz dreimal intraperitoneal mit 20µg DB im Abstand von 4 Wochen immunisiert. Vollblut der Tiere wurden nach 12 bzw. 32 Wochen nach der ersten Injektion durch Herzpunktion nach Ethernarkose gewonnen, 2 Std bei 37°C zur Agglutination inkubiert, über Nacht bei 4°C gelagert und am nächsten Tag 10 min bei 14.000 Upm abzentrifugiert. Der klare Serumüberstand wurde abgezogen und in kleinen Aliquots bei -20°C gelagert. In diesen Seren wurde der Gehalt an HCMV-spezifischen Immunglobulinen der Subklassen IgG1 und IgG2a bestimmt. Während einer Th1-typischen Immunantwort werden bevorzugt Antikörper der Klasse IgG2a gebildet, während bei einer Th2-typischen Immunantwort Antikörper der Klasse IgGl überwiegen. Seren der Versuchstiere wurden 30 min bei 56°C inaktiviert und mit Hilfe eines kommerziell verfügbaren ELISA, beschichtet mit den Antigenen extrazellulärer HCMV-Partikel (Biotest AG, Dreieich) getestet. Dazu wurden serielle Verdünnungsreihen der Testseren hergestellt, in die Vertiefungen der ELISA-Platte pipettiert und 2 Std bei 37°C inkubiert. Nach 3 Waschschritten in PBS/0,05% Tween 20 wurde der Subklasse-spezifische, HRPgekoppelte Sekundärantikörper in einer Verdünnung von 1:2000 in PBS/2% Tween 20/3% FKS zugegeben und 1 Std bei 37°C und feuchter Atmosphäre inkubiert. Nach 4 weiteren Waschschritten schloss sich eine OPD-Färbereaktion an, die durch Zugabe von 100 µl 0,5M H₂SO₄ gestoppt wurde. Danach wurde die Absorption bei einer Wellenlänge von 492 nm in einem ELISA-Reader gemessen.

Durch die Absorptionswerte der verschiedenen Verdünnungen eines Serums wurde eine Regresionsgerade gelegt und derjenige Wert, der der 2,5-fachen Absorption eines Nullserums entsprach, als sog. "Endpunktverdünnung" ermittelt. Aus dem Verhältnis der Endpunktverdünnungen für IgG1:IgG2a ergab sich ein Quotient,der unabhängig vom Immunisierungsprotokoll in jedem Fall unter 0,3 lag (Tabelle 1). Da IgG1:IgG2a-Quotienten von <1 für eine Th1-typische Immunantwort sprechen, während Quotienten >1 auf eine TH2-Antwort hinweisen, beweist dieses Experiment, dass die Immunisierung mit DB eine Th1-artige T-Helferzellantwort induziert. Analoge Ergebnisse wurden in einem parallelen Versuch erzielt, bei dem DB intraplantar appliziert wurden. Dies zeigt, daß, unabhängig von der Applikationsform, die Immunisierung mit DB zu einer Th1-typischen Immunantwort der Versuchstiere führt.

### Beispiel 7 :

### Technik zur Expression von heterologen Genen in primären humanen Fibroblasten und Komplementation von Defektmutanten des HCMV in solchen Zellen

Humane Fibroblasten in möglichst niedriger Passage werden auf 6-well Platten ausgesät, wobei jeweils 10⁵ Zellen eingesetzt werden. In einige der Vertiefungen wird vorher zu Kontrollzwecken ein Deckgläschen eingebracht.

Die Zellen werden über Nacht in einem CO₂-Brutschrank bei 37°C inkubiert und am nächsten Tag - transfiziert. Das gewünschte Transgen wird beispielsweise auf dem Plasmid pcDNA6/V5-His (Invitrogen) exprimiert, welches gleichzeitig das Blasticidin-S-Deaminase Gen trägt. Expression dieses Gens vermittelt Resistenz gegen die Substanz Blasticidin S.

Alternativ kann das gewünschte Transgen auf einem separaten Expressionsplasmid zusammen mit dem Vektor pRG273, der Resistenz gegen das Antibiotikum Puromycin vermittelt, transfiziert werden. Dieser Vektor ist ein Abkömmling des Vektors pLXSN16E6E7 und exprimiert neben dem Resistenzgen die transformierenden Proteine E6 und E7 von Humanem Papillomvirus 16. Dies kann durch Transformation der getroffenen Zellen deren Wachstumspotenz erhöhen.

Die Transfektion wird mit der "Fugene-Methode" der Fa. Roche Diagnostics druchgeführt. Hierzu werden 3µl Fugene-Reagenz mit 97 µl Medium ohne FKS gemischt und 5 Minuten bei Raumtemperatur inkubiert. Dann werden ca. 2,5 µg DNA des Transgen-Plasmids (bei Doppeltransfektionen zusätzlich 50 ng von pRG273) zugegeben, nach weiteren 15 Minuten bei Raumtemperatur werden 900 µl serumhaltiges Medium zugesetzt. Diese Mischung wird nach Entfernen des alten Mediums zu den Zellen gegeben. Die Transfektion erfolgt für 3-8 Stunden. Danach wird der Transfektionsansatz entfernt und Kulturmedium zugegeben. Nach 2 Tagen Kultivierung werden die Deckgläschen in 24-Well Platten überführt, gewaschen und mit 90%igem Aceton fixiert. Mit Hilfe von Antikörpern wird die Expression des Transgens durch indirekte Immunfluoreszenzmikroskopie überprüft.

Bei guter Transfektionseffizienz werden die restlichen Zellen abtrypsiniert und in Selektionsmedium ausgesät (z.B. 3 µg/ml Blasticidin, 2µg/ml Puromycin, 250 µg/ml G418 usw). Wenn sich im Selektionsmedium Zellkolonien bilden, werden diese isoliert und unter Selektionsbedingungen weiterkultiviert. Die Zellen werden in der Folge unter Selektionsbedingungen expandiert und die Expressionsstärke des Transgens z.B. im Immunblot überprüft. Bei guter Expression werden die Zellen aliquotiert und in flüssigem Stickstoff bis zur weiteren Verwendung gelagert.

### Beispiel 8:

### Strategie zur Generierung von rekombinanten DB, die Fusionsproteine von pp65 mit homologen oder heterologen Antigenen enthalten

Zur Herstellung rekombinanter DB kann nach folgender Strategie vorgegangen werden:

Ausgehend von dem genomischen DNA-Fragment BamHI-R (kloniert in pBluescribe als pBamHIR) des Stammes Ad169 werden zunächst Plasmidklone hergestellt, die heterologe Nukleinsäure-Abschnitte in eine singuläre SrfI-Schnittstelle im 3'-Anteil des pp65-Gens einkloniert enthalten. Es werden die kodierenden DNA-Regionen für verschiedene antigene Abschnitte von anderen HCMV-Proteinen als pp65, bzw. von Proteinen anderer Erreger in einer Weise kloniert, daß ein durchgängiger offener Leserahmen mit UL83 (pp65) entsteht. In 3'-Richtung zu diesen heterologen DNA-Abschnitten kann, ebenfalls in dem durchgängigen Leserahmen, ein Fusionsgen, bestehend aus dem Gen für das sogenannte Green-fluorescent Protein (GFP) und der Neomycin-Transferase kloniert werden. Dieses Gen soll durch jeweils eine Erkennungsstelle für die Cre-Rekombinase (LoxP-Stellen) des Phagen P1 flankiert werden. Die dabei entstehenden Plasmidkonstrukte sind in Abbildung 7 dargestellt.

Die Rekombinationsplasmide werden in humane Vorhaut-Fibroblasten transfiziert. Diese Zellen werden mit dem HCMV-Stamm Ad169 überinfiziert. Durch homologe Rekombination entstehen rekombinante HCMV-Genommoleküle, die das jeweilige Fusionsgen anstelle von pp65 enthalten. Durch Zugabe von G418 zum Kulturmedium wird auf die Vermehrung dieser rekombinanten DNA-Moleküle und auf die Anreicherung rekombinanter Viren selektioniert. Durch die Expression von GFP besteht darüber hinaus die Möglichkeit, solche Zellen mit Hilfe der Zytofluorometrie (FACS) positiv zu sortieren, die rekombinante virale Genome enthalten. Auf diese Weise ist eine effiziente Anreicherung und nachfolgende Isolierung rekombinanter Viren gewährleistet.

Die aufgereinigten Viren werden dann über Zellinien passagiert, welche transient oder stabil die CRE-Rekombinase exprimieren. Diese Rekombinase erkennt in einem DNA-Molekül eine kurze Abfolge von Basenpaaren (loxP-Stellen), und schneidet punktgenau die zwischen solchen loxP-Stellen gelegenen DNA-Abschnitte aus. Dies hat zur Folge, daß die für die Selektion verwendeten DNA-Abschnitte, die für GFP-Neo kodieren, entfernt werden. Durch Selektion GFP-negativer Zellen im FACS wird in der Folge reines Virus, welches ein pp65-Fusionsprotein für die Selektion exprimiert, isoliert und gereinigt.

Reine Viruspopulationen können in der Folge dazu eingesetzt werden, humane Fibroblasten zu infizieren. In diesen infizierten Zellen entstehen im Rahmen der Virusinfektion rekombinante DB, die in der Folge aus dem Zellkultur-Überstand wiederum mittels Gradientenzentrifugation gereinigt werden können.

Es können beispielsweise folgende Viren hergestellt werden (siehe Abb. 7):
A) Viren, welche ein Fusionsprotein bestehend aus pp65 und bekannten, neutralisierenden Domänen aus gB von HCMV exprimieren. Derartige Domänen wurden in der Literatur als AD1 und AD2 bezeichnet. Die homologe Domäne zu AD2 aus dem gB des Laborstammes Towne wurde mit TW2 bezeichnet.
B) Viren, welche ein Fusionsprotein bestehend aus pp65 und bekannten CTL-Epitopen des IE1-Proteins (IE1) von HCMV exprimieren.
C) Viren, welche ein Fusionsprotein bestehend aus pp65, aus bekannten neutralisierenden Domänen von HCMV-Glykoprotein (NA) sowie aus bekannten CTL-Epitopen des IE1-Proteins (IE1) von HCMV exprimieren.
D) Viren, welche ein Fusionsprotein bestehend aus pp65 und aus bekannten CTL-Epitopen anderer Erreger, wie beispielsweise aus dem Nukleoprotein von Influenza A Virus (z.B. AS 147-155) exprimieren.
E) Viren, welche ein Fusionsprotein bestehend aus pp65, aus bekannten neutralisierenden Epitopen (NA) eines anderen Erregers und aus bekannten CTL-Epitopen (CTL) dieses anderen Erregers exprimiert.

## Patentansprüche

1. Virale Partikel, die nach Infektion von Säugerzellen durch humanes Cytomegalovirus (HCMV) freigesetzt werden, **dadurch gekennzeichnet,**
a) **daß** die Partikel von einer Lipidmembran umgeben sind, in die virale HCMV-Glykoproteine eingelagert sind,
b) **daß** die Partikel weder virale DNA noch Capside enthalten,
c) **daß** die Partikel ein Fusionsprotein enthalten, umfassend einen oder mehrere Teile des T-Zellantigens pp65 (UL83) und einen oder mehrere Teile eines oder mehrerer Proteine, die nicht pp65 sind,
wobei die Partikel eine hohe Antigenität aufweisen und die Bildung neutralisierender Antikörper induzieren können.

2. Partikel nach Anspruch 1, **dadurch gekennzeichnet, daß** das T-Zellantigen pp65 (UL83) mit einem oder mehreren Teilen eines Glykoproteins des HCMV fusioniert ist.

3. Partikel nach Anspruch 1, **dadurch gekennzeichnet, daß** das T-Zellantigen pp65 (UL83) mit einem oder mehreren Teilen des HCMV-Glycoproteins gB fusioniert ist.

4. Partikel nach Anspruch 1, **dadurch gekennzeichnet, daß** das T-Zellantigen pp65 (UL83) mit einem oder mehreren Teilen des HCMV-Glycoproteins gH fusioniert ist.

5. Partikel nach Anspruch 1, **dadurch gekennzeichnet, daß** das T-Zellantigen pp65 (UL83) mit einem oder mehreren Teilen des HCKV-Proteins IE1 (ppUL123) fusioniert ist.

6. Partikel nach Anspruch 1, **dadurch gekennzeichnet, daß** das T-Zellantigen pp65 (UL83) mit einem oder mehreren Teilen eines Glykoproteins des HCMV und mit einem oder mehreren Teilen des HCMV-Proteins IE1 (ppUL123) fusioniert ist.

7. Partikel nach Anspruch 1, **dadurch gekennzeichnet, daß** das T-Zellantigen pp65 (UL83) mit einem oder mehreren Teilen eines Proteins fusioniert ist, das Teil eines anderen humanpathogenen Erregers als HCMV ist.

8. Partikel nach Anspruch 7, **dadurch gekennzeichnet, daß** gegen das Protein, das Teil eines anderen humanpathogenen Erregers als HCMV ist, bei natürlicher Infektion mit dem Erreger im Menschen cytotoxische T-Lymphocyten (CTL) gebildet werden.

9. Partikel nach Anspruch 8, **dadurch gekennzeichnet, daß** der andere humanpathogene Erreger, der nicht HCMV ist, ausgewählt ist aus der Gruppe umfassend HIV-1, HBV, HCV und Influenza.

10. Partikel nach Anspruch 8, **dadurch gekennzeichnet, daß** das Fusionsprotein wenigstens ein Epitop eines Proteins des anderen humanpathogenen Erregers umfaßt, wobei gegen das Epitop bei Infektion im Menschen neutralisierende Antikörper gebildet werden, und wenigstens ein weiteres Epitop eines Proteins des anderen humanpathogenen Erregers, wobei gegen das weitere Epitop bei Infektion im Menschen CTL gebildet werden.

11. Partikel nach einem der Ansprüche 1-9, **dadurch gekennzeichnet, daß** das Fusionsprotein wenigstens ein Epitop umfaßt, gegen das bei Infektion im Menschen neutralisierende Antikörper gebildet werden, und wenigstens ein weiteres Epitop, gegen das bei Infektion im Menschen CTL gebildet werden, wobei die Epitope von Proteinen des gleichen Erregers abgeleitet sind.

12. Virale Partikel, die nach Infektion von Säugerzellen durch HCMV freigesetzt werden, **dadurch gekennzeichnet,**
a) **daß** die Partikel von einer Lipidmembran umgeben sind, in die virale HCMV-Glykoproteine eingelagert sind,
b) **daß** die Partikel weder virale DNA noch Capside enthalten,
c) **daß** die Partikel Teile von mindestens 2 HCMV-Glykoproteinen enthalten, die Varianten eines bestimmten Glykoproteins von verschiedenen Stämmen des HCMV sind,
wobei die Partikel eine hohe Antigenität aufweisen und die Bildung neutralisierender Antikörper induzieren können.

13. Partikel nach Anspruch 12, **dadurch gekennzeichnet, daß** von den 2 varianten des bestimmten HCMV-Glykoproteins eine die Variante des HCMV-Stammes Towne, die andere die Variante des HCMV-Stammes Ad 169 ist.

14. Partikel nach Anspruch 12, **dadurch gekennzeichnet, daß** das Glykoprotein das Protein gB des HCMV ist.

15. Verfahren zur Vermehrung von HCMV, das folgende Schritte umfaßt:
a) Bereitstellung eines HCMV, in dessen Genom ein essentielles Gen deletiert worden ist, so dass das HCMV nicht mehr vermehrungsfähig ist,
b) Bereitstellung einer stabil transfizierten Säugerzellinie, die das in a) deletierte Gen des HCMV exprimiert,
c) Vermehrung des deletierten Virus aus a) in Zellen gemäß b).

16. Verfahren nach Anspruch 15, **dadurch gekennzeichnet, daß** in Schritt b) humane Vorhautfibroblasten transfiziert werden.

17. Verfahren nach Anspruch 15, **dadurch gekennzeichnet, daß** die Säugerzellen mit Hilfe eines lipidhaltigen Reagenz transfiziert werden.

18. Verfahren nach Anspruch 15, **dadurch gekennzeichnet, daß** die Säugerzellen durch das "Fugene"-Reagenz transfiziert werden.

19. Verfahren nach Anspruch 15, **dadurch gekennzeichnet, daß** in Schritt a) das HCMV eine Deletion im Gen des Hauptcapsidproteins (UL86) trägt.

20. Verfahren zur Herstellung viraler Partikel, das folgende Schritte umfaßt:
a) Bereitstellung von HCMV gemäß einem Verfahren der Ansprüche 15-19,
b) Infektion von Säugerzellen mit Virus, das gemäß Schritt a) vermehrt wurde,
c) Isolierung von viralen Partikeln aus Zellen, die gemäß Schritt b) infiziert wurden, wobei
α) die Partikel von einer Lipidmembran umgeben sind, in die virale HCMV-Glykoproteine eingelagert sind,
β) die Partikel weder virale DNA noch Capside enthalten.

21. Verwendung viraler Partikel, die nach Infektion von Säugerzellen durch humanes Cytomegalovirus (HCMV) freigesetzt werden, zur Herstellung eines Impfstoffes **dadurch gekennzeichnet,**
a) **daß** die Partikel von einer Lipidmembran umgeben sind, in die virale HCMV-Glykoproteine eingelagert sind,
b) **daß** die Partikel weder virale DNA noch Capside enthalten.

22. Verwendung nach Anspruch 21, **dadurch gekennzeichnet, daß** die viralen Partikel weiterhin ein Fusionsprotein enthalten, umfassend einen oder mehrere Teile des T-Zellantigens pp65 (UL83) und einen oder mehrere Teile eines oder mehrerer Proteine, die nicht pp65 sind.

23. Verwendung nach Anspruch 21, **dadurch gekennzeichnet, daß** die Partikel Teile von mindestens 2 Glykoproteinen enthalten, die Varianten eines bestimmten Glykoproteins von verschiedenen Stämmen des HCMV sind.

24. Verwendung nach Anspruch 21, **dadurch gekennzeichnet, daß** die viralen Partikel durch ein Verfahren gemäß Anspruch 20 hergestellt worden sind.

## Claims

1. Viral particles released after infection of mammalian cells by human cytomegalovirus (HCMV), **characterized**
a) **in that** the particles are surrounded by a lipid membrane in which viral HCMV glycoproteins are embedded,
b) **in that** the particles contain neither viral DNA nor capsids,
c) **in that** the particles contain a fusion protein comprising one or more parts of the T-cell antigen pp65 (UL83) and one or more parts of one or more proteins which are not pp65,
wherein the particles have a high antigenicity and can induce the formation of neutralizing antibodies.

2. Particles as claimed in claim 1, **characterized in that** the T-cell antigen pp65 (UL83) is fused to one or more parts of an HCMV glycoprotein.

3. Particles as claimed in claim 1, **characterized in that** the T-cell antigen pp65 (UL83) is fused to one or more parts of the HCMV glycoprotein gB.

4. Particles as claimed in claim 1, **characterized in that** the T-cell antigen pp65 (UL83) is fused to one or more parts of the HCMV glycoprotein gH.

5. Particles as claimed in claim 1, **characterized in that** the T-cell antigen pp65 (UL83) is fused to one or more parts of the HCMV protein IE1 (ppUL123).

6. Particles as claimed in claim 1, **characterized in that** the T-cell antigen pp65 (UL83) is fused to one or more parts of an HCMV glycoprotein and to one or more parts of the HCMV protein IE1 (ppUL123).

7. Particles as claimed in claim 1, **characterized in that** the T-cell antigen pp65 (UL83) is fused to one or more parts of a protein which is part of a human pathogen other than HCMV.

8. Particles as claimed in claim 7, **characterized in that** cytotoxic T lymphocytes (CTL) are formed in humans against the protein which is part of a human pathogen other than HCMV on natural infection with the pathogen.

9. Particles as claimed in claim 8, **characterized in that** the other human pathogen which is not HCMV is selected from the group comprising HIV-1, HBV, HCV and influenza.

10. Particles as claimed in claim 8, **characterized in that** the fusion protein comprises at least one epitope of a protein of the other human pathogen, neutralizing antibodies against the epitope being formed in humans on infection, and at least one other epitope of a protein of the other human pathogen, CTL against the other epitope being formed in humans on infection.

11. Particles as claimed in any of claims 1 to 9, **characterized in that** the fusion protein comprises at least one epitope against which neutralizing antibodies are formed in humans on infection, and at least one other epitope against which CTL are formed in humans on infection, the epitopes being derived from proteins of the same pathogen.

12. Viral particles released after infection of mammalian cells by HCMV, **characterized**
a) **in that** the particles are surrounded by a lipid membrane in which viral HCMV glycoproteins are embedded,
b) **in that** the particles contain neither viral DNA nor capsids,
c) **in that** the particles contain parts of at least 2 HCMV glycoproteins which are variants of a particular glycoprotein from different HCMV strains,
wherein the particles have a high antigenicity and can induce the formation of neutralizing antibodies.

13. Particles as claimed in claim 12, **characterized in that** one of the 2 variants of the particular HCMV glycoprotein is the variant of the HCMV Towne strain and the other is the variant of the HCMV Ad169 strain.

14. Particles as claimed in claim 12, **characterized in that** the glycoprotein is the gB protein of HCMV.

15. A method for replicating HCMV which comprises the following steps:
a) provision of an HCMV in whose genome an essential gene has been deleted, so that the HCMV is no longer able to replicate,
b) provision of a stably transfected mammalian cell line which expresses the HCMV gene deleted in a),
c) replication of the deleted virus from a) in cells from b).

16. The method as claimed in claim 15, **characterized in that** human foreskin fibroblasts are transfected in step b).

17. The method as claimed in claim 15, **characterized in that** the mammalian cells are transfected with the aid of a lipid-containing reagent.

18. The method as claimed in claim 15, **characterized in that** the mammalian cells are transfected by the "Fugene" reagent.

19. The method as claimed in claim 15, **characterized in that** the HCMV in step a) harbors a deletion in the gene of the major capsid protein (UL86).

20. A method for producing viral particles which comprises the following steps:
a) provision of HCMV as set forth in the method of any of claims 15-19,
b) infection of mammalian cells with virus which has been replicated as in step a),
c) isolation of viral particles from cells which have been infected as in step b), where
α) the particles are surrounded by a lipid membrane in which viral HCMV glycoproteins are embedded,
β) the particles contain neither viral DNA nor capsids.

21. The use of viral particles released after infection of mammalian cells by human cytomegalovirus (HCMV) for the preparation of a vaccine, **characterized**
a) **in that** the particles are surrounded by a lipid membrane in which viral HCMV glycoproteins are embedded,
b) **in that** the particles contain neither viral DNA nor capsids.

22. The use as claimed in claim 21, **characterized in that** the viral particles additionally contain a fusion protein comprising one or more parts of the T-cell antigen pp65 (UL83) and one or more parts of one or more proteins which are not pp65.

23. The use as claimed in claim 21, **characterized in that** the particles contain parts of at least 2 glycoproteins which are variants of a particular glycoprotein from different HCMV strains.

24. The use as claimed in claim 21, **characterized in that** the viral particles have been produced by a method as claimed in claim 20.

## Revendications

1. Particules virales, qui sont libérées après infection de cellules mammifères par le cytomégalovirus humain (HCMV), **caractérisées en ce que** :
a) les particules sont enveloppées d'une membrane lipidique, dans laquelle sont stockées des glycoprotéines virales du HCMV,
b) les particules ne contiennent ni ADN viral ni capside,
c) les particules contiennent une protéine de fusion, comprenant une ou plusieurs parties de l'antigène pp65 (UL83) du lymphocyte T et une ou plusieurs parties d'une ou de plusieurs protéines différentes de pp65,
les particules pouvant présenter une antigénicité élevée et induire la formation d'anticorps de neutralisation.

2. Particules selon la revendication 1, **caractérisées en ce que** l'on fusionne l'antigène pp65 (UL83) du lymphocyte T avec une ou plusieurs parties d'une glycoprotéine du HCMV.

3. Particules selon la revendication 1, **caractérisées en ce que** l'on fusionne l'antigène pp65 (UL83) du lymphocyte T avec une ou plusieurs parties de la glycoprotéine gB du HCMV.

4. Particules selon la revendication 1,
**caractérisées en ce que** l'on fusionne l'antigène pp65 (UL83) du lymphocyte T avec une ou plusieurs parties de la glycoprotéine gH du HCMV.

5. Particules selon la revendication 1, **caractérisées en ce que** l'on fusionne l'antigène pp65 (UL83) du lymphocyte T avec une ou plusieurs parties de la protéine IE1 (ppUL123) du HCMV.

6. Particules selon la revendication 1, **caractérisées en ce que** l'on fusionne l'antigène pp65 (UL83) du lymphocyte T avec une ou plusieurs parties d'une glycoprotéine du HCMV et avec une ou plusieurs parties de la protéine IE1 (ppUL123) du HCMV.

7. Particules selon la revendication 1, **caractérisées en ce que** l'on fusionne l'antigène pp65 (UL83) du lymphocyte T avec une ou plusieurs parties d'une protéine appartenant à un autre agent pathogène pour l'être humain, différent du HCMV.

8. Particules selon la revendication 7, **caractérisées en ce que** des lymphocytes T cytotoxiques (CTL) sont formés contre la protéine appartenant à un autre agent pathogène pour l'être humain, différent du HCMV, lors d'une infection naturelle avec l'agent pathogène pour l'être humain.

9. Particules selon la revendication 8, **caractérisées en ce que** l'on choisit l'autre agent pathogène pour l'être humain, différent du HCMV, dans le groupe constitué des virus VIH-1, VHB, VHC et de la grippe.

10. Particules selon la revendication 8, **caractérisées en ce que** la protéine de fusion comprend au moins un épitope d'une protéine provenant de l'autre agent pathogène pour l'être humain, des anticorps de neutralisation étant formés contre l'épitope lors d'une infection chez l'être humain, et au moins un autre épitope d'une protéine provenant de l'autre agent pathogène pour l'être humain, des CTL étant formés contre l'autre épitope lors d'une infection chez l'être humain.

11. Particules selon l'une quelconque des revendications 1 à 9, **caractérisées en ce que** la protéine de fusion comprend au moins un épitope, contre lequel se sont formés des anticorps de neutralisation lors d'une infection chez l'être humain, et au moins un autre épitope, contre lequel se sont formés des CTL lors d'une infection chez l'être humain,
les épitopes dérivant de protéines du même agent pathogène.

12. Particules virales, qui sont libérées après infection de cellules mammifères par le cytomégalovirus humain (HCMV), **caractérisées en ce que** :
a) les particules sont enveloppées d'une membrane lipidique, dans laquelle sont stockées des glycoprotéines virales du HCMV,
b) les particules ne contiennent ni ADN viral ni capside,
c) les particules contiennent des parties d'au moins 2 glycoprotéines du HCMV, qui sont des variantes d'une glycoprotéine déterminée de différentes souches du HCMV,
les particules pouvant présenter une antigénicité élevée et induire la formation d'anticorps de neutralisation.

13. Particules selon la revendication 12, **caractérisées en ce que**, sur les 2 variantes de la glycoprotéine du HCMV déterminée, l'une est la variante Towne de la souche HCMV et l'autre est la variante Ad 169 de la souche HCMV.

14. Particules selon la revendication 12, **caractérisées en ce que** la glycoprotéine est la protéine gB du HCMV.

15. Procédé de multiplication du HCMV, comprenant les étapes suivantes :
a) la préparation d'un HCMV, dont le génome a été délété d'un gène essentiel, de sorte que le HCMV ne puisse plus se multiplier,
b) la préparation d'une lignée de cellules mammifères transfectée de manière stable, qui exprime le gène du HCMV délété de l'étape a),
c) la multiplication du virus délété issu de l'étape a) dans des cellules conformes à l'étape b).

16. Procédé selon la revendication 15, **caractérisé en ce que** l'on transfecte des fibroblastes de prépuce humain à l'étape b).

17. Procédé selon la revendication 15, **caractérisé en ce que** l'on transfecte les cellules mammifères à l'aide d'un réactif contenant des lipides.

18. Procédé selon la revendication 15, **caractérisé en ce que** l'on transfecte les cellules mammifères par le biais du réactif "Fugene".

19. Procédé selon la revendication 15, **caractérisé en ce que** le HCMV porte, à l'étape a), une délétion dans le gène de la protéine de capside principale (UL86).

20. Procédé pour produire des particules virales, comprenant les étapes suivantes :
a) la préparation du HCMV selon un procédé conforme aux revendications 15 à 19,
b) l'infection de cellules mammifères par un virus qui a été multiplié selon l'étape a),
c) l'isolement de particules virales à partir de cellules qui ont été infectées selon l'étape b), où
α) les particules sont enveloppées d'une membrane lipidique dans laquelle sont stockées des glycoprotéines virales du HCMV,
β) les particules ne contiennent ni ADN viral ni capside.

21. Utilisation de particules virales, qui ont été libérées après infection de cellules mammifères par un cytomégalovirus humain (HCMV) pour produire un vaccin, **caractérisée en ce que** :
a) les particules sont enveloppées d'une membrane lipidique dans laquelle sont stockées des glycoprotéines virales du HCMV,
b) les particules ne contiennent ni ADN viral ni capside.

22. Utilisation selon la revendication 21, **caractérisée en ce que** les particules virales contiennent en outre une protéine de fusion, comprenant une ou plusieurs parties de l'antigène pp65 (UL83) du lymphocyte T et une ou plusieurs parties d'une ou de plusieurs protéines différentes de pp65.

23. Utilisation selon la revendication 21, **caractérisée en ce que** les particules contiennent des parties d'au moins 2 glycoprotéines qui sont des variantes d'une glycoprotéine déterminée de différentes souches du HCMV.

24. Utilisation selon la revendication 21, **caractérisée en ce que** les particules virales sont produites par un procédé selon la revendication 20.
